# EUROPÄISCHE PATENTANMELDUNG

(11) **EP 4 254 431 A2**
(43) Veröffentlichungstag der Anmeldung: **04.10.2023**
(21) Anmeldenummer: 22195944.8
(22) Anmeldetag: 15.09.2022
(51) Int. Cl.: G21F 3/00, G21F 7/02

(54) **STRAHLENSCHUTZVORRICHTUNG ZUR ABSCHIRMUNG IONISIERENDER STRAHLUNG**

(30) Priorität: 31.03.2022 DE 102022203183
(71) Anmelder: Siemens Healthcare GmbH, 91052 Erlangen (DE)
(72) Erfinder: Riess, Steffen, 90610 Winkelhaid (DE); Reichert, Armin, 90547 Stein (DE); Ruf, Marcel, 91094 Langensendelbach (DE)

(57) **Zusammenfassung**

Offenbart wird eine Strahlenschutzvorrichtung (200) zur Abschirmung eines Nutzers (N) vor ionisierender Strahlung. Die Strahlenschutzvorrichtung (200) weist ein Glaselement (210), das als Schutzglas gegen die ionisierende Strahlung ausgebildet ist, eine Interaktionsvorrichtung (220) zum Empfangen einer Nutzereingabe durch den Nutzer (N) und eine Freigabevorrichtung (270) zum Empfangen einer Freigabeeingabe durch den Nutzer (N)

## Beschreibung

Die Erfindung betrifft eine Strahlenschutzvorrichtung zur Abschirmung von ionisierender Strahlung, die insbesondere von einer medizinischen bildgebenden Modalität ausgesandt wird. Insbesondere betrifft die Erfindung eine Strahlenschutzvorrichtung zum Empfangen einer Nutzereingabe durch einen Nutzer. Ferner betrifft die Erfindung eine bildgebende Modalität mit der Strahlenschutzvorrichtung.

Bildgebende Modalitäten emittieren im Betrieb ionisierende Strahlung, wie etwa Röntgenstrahlung. Nutzer bildgebender Modalitäten, wie etwa Arzt- oder Bedienpersonal, müssen sich im Betrieb vor der ionisierenden Strahlung schützen. Dies kann etwa dadurch erreicht werden, dass sich Nutzer im Betrieb in einem gegen ionisierende Strahlung abgeschirmten Raum aufhalten.

Für viele Anwendungen ist allerdings die Anwesenheit des Nutzers in der direkten Umgebung der bildgebenden Modalität notwendig, etwa bei klassischen Röntgenuntersuchungen, um Geräteeinstellungen anzupassen, bei interventioneller Radiologie, bei der ein Eingriff unter kontinuierlicher Röntgen-Durchlichtkontrolle stattfindet, oder bei Angiografieanwendungen. Vor allem wenn die medizinische Modalität kontinuierlich eingesetzt wird, etwa um dem Nutzer ein Röntgenbild als Live-Bildgebung bereitzustellen, sind aufwändigere Strahlenschutzmaßnahmen zu treffen, da der Nutzer aus Gründen des Workflows nicht bei jedem "Schuss" den Raum verlassen kann.

In diesem Umfeld werden optisch transparente Scheiben aus z.B. Bleiglas oder Bleiacryl eingesetzt, die bzgl. der Röntgenstrahlung eine hohe Schirmwirkung besitzen. Das Bedienpersonal kann sich hinter derartigen Strahlenschutzscheiben aufhalten und nach wie vor auf den Patienten blicken, ist jedoch vor Streustrahlung geschützt.

Eine Schwierigkeit bei derartigen Prozeduren unter kontinuierlicher Durchlichtkontrolle besteht ferner in einem vergleichsweise hohen operativen Aufwand seitens des Nutzers. Neben der Beobachtung des Patienten durch die Strahlenschutzscheibe muss der Nutzer die bildgebende Modalität oder den Eingriff selbst steuern und entsprechende Informationen sichten und berücksichtigen. Dies wird zudem dadurch erschwert, dass der Nutzer hinter der Strahlenschutzscheibe in seiner Bewegungsfreiheit eingeschränkt ist und z.B. in einem Untersuchungsraum nicht ohne weiteres zu einem Terminal der bildgebenden Modalität wechseln kann.

Eine Benutzerinteraktion mit bildgebenden Modalitäten und/oder Modalitäten, die ionisierende Strahlung emittieren, kann mittels Eingabemedien erfolgen, beispielsweise mittels Knöpfe, Joysticks, kapazitiven und/oder resistive Touchdisplays. Konventionelle Druckknöpfe und Joysticks verfügen über ein haptisches Feedback, sodass der Benutzer intuitiv durch die Bewegung des Joysticks oder das Drücken des Knopfes bemerkt, dass seine Eingabe erkannt wurde. Das Feedback bzw. die Rückmeldung signalisiert dem Benutzer z.B. ein Auslösen eines Befehls und/oder ein Erreichen einer Schaltschwelle.

In transparente und/oder flächige Vorrichtungen, wie beispielsweise transparente Strahlenschutzvorrichtungen sind die aus dem Stand der Technik bekannten Schalter, Knöpfe und/oder Joysticks nicht ohne weiteres bzw. ohne in Kauf nehmen von Rückschritten verwendbar.

Für funktional sichere Bedienelemente, zum Beispiel zur Bewegungssteuerung oder zur Strahlungsauslösung, sind im Bereich der Medizintechnik besondere Anforderungen zu berücksichtigen. Für zulassungskonforme Umsetzungen sind insbesondere Aspekte der "Control/Protect-Pfad-Architektur" zu berücksichtigen. Bedienelemente, die insbesondere zusammen mit einer flächigen und/oder transparenten Vorrichtung, sollen zum einen nahe an den Nutzer gebracht werden und zum anderen zur Triggerung von Vorgängen (z. B. Tischbewegung, Strahlungsauslösung) funktional gesichert sein.

Vor diesem Hintergrund ist es eine Aufgabe der Erfindung, eine Vorrichtung bereitzustellen, die eine verbesserte Kontrolle und/oder Steuerung von Prozessen unter Verwendung eines eine ionisierende Strahlung aussendenden Geräts durch einen Nutzer ermöglicht und den Nutzer gleichzeitig vor ionisierender Strahlung schützt. Ferner ist es Aufgabe der Erfindung, die Bedienung und/oder Steuerung einer medizinischen, bildgebenden Modalität von einem strahlungsgeschützten Bereich durchführen zu können, wobei die Steuerung und/oder die Modalität vom strahlungsgeschützten Bereich mindestens teilweise sichtbar ist und die Bedienung/Steuerung funktional abgesichert ist.

Die Aufgabe wird gelöst durch eine Strahlenschutzvorrichtung sowie einer medizinischen bildgebenden Vorrichtung gemäß dem Hauptanspruch und den nebengeordneten Ansprüchen. Vorteilhafte und/oder bevorzugte Ausführungsformen ergeben sich aus den Unteransprüchen, der Beschreibung und den beigefügten Figuren.

Gemäß einem Aspekt wird eine Strahlenschutzvorrichtung zur Abschirmung eines Nutzers vor ionisierender Strahlung bereitgestellt. Die Strahlenschutzvorrichtung umfasst ein, insbesondere flächiges, Glaselement, das als Schutzglas gegen die ionisierende Strahlung ausgebildet ist, und eine, insbesondere flächige, Interaktionsvorrichtung zum Empfangen einer Nutzereingabe durch den Nutzer. Optional ist die Interaktionsvorrichtung zum Darstellen von Daten für den Nutzer ausgebildet. Insbesondere ist die Interaktionsvorrichtung wenigstens abschnittsweise transparent oder transluzent ausgebildet. Die Interaktionsvorrichtung überdeckt vorzugsweise wenigstens einen Teil einer Fläche des Glaselements.

"Transparent ausgebildet" kann in diesem Zusammenhang insbesondere heißen, dass die Interaktionsvorrichtung wenigstens abschnittsweise eine Transmission von wenigstens 80 % für Wellenlängen im sichtbaren Bereich von 420 nm bis 780 nm aufweist.

Das Glaselement ist vorzugsweise mindestens abschnittsweise, im Speziellen vollständig, transparent oder transluzent ausgebildet. Das Glaselement kann ebenfalls wenigstens abschnittsweise eine Transmission von wenigstens 80 % für Wellenlängen im sichtbaren Bereich von 420 nm bis 780 nm aufweisen.

Die ionisierende Strahlung kann insbesondere Röntgenstrahlung umfassen, wie sie z.B. von einem mit Röntgenstrahlung operierenden Gerät, wie einem C-Bogen oder einem Computertomographiegerät, emittiert wird. Insbesondere kann das Glaselement als Röntgenschutzglas ausgebildet sein. Insbesondere kann das Glaselement ein Bleiacrylglas umfassen. Insbesondere kann das Glaselement in der Glaszusammensetzung einen Bleigehalt größer oder gleich 60 Gew.-%, bevorzugt größer 60 Gew.-%, insbesondere bevorzugt größer oder gleich 65 Gew.-%, weiter bevorzugt größer oder gleich 70 Gew.-%, umfassen.

Ferner kann das Glaselement im Bereich einer Röntgenspannung von 40 kV - 200 kV einer Röntgenröhre einen Bleigleichwert von 0,5 mm - 7 mm aufweisen.

Die ionisierende Strahlung kann dabei von einem Gerät emittiert werden. Der Nutzer kann insbesondere ein Nutzer des Geräts sein.

Die Interaktionsvorrichtung ist vorzugsweise flächig ausgebildet. Beispielsweise umfasst oder bildet die Interaktionsvorrichtung eine berührungsempfindliche Schicht, insbesondere eine transparente und/oder transluzente berührungsempfindliche Schicht. Im Speziellen ist die Interaktionsvorrichtung flächig ausgebildet zum Darstellen von Daten auf einer Fläche. Vorzugsweise ist die Interaktionsvorrichtung während der Darstellung von Daten semi-transparent und/oder transluzent, sodass hinter der Ebene der Interaktionsvorrichtung liegende Objekte mindestens schemenhaft erkennbar sind. Insbesondere kann die Interaktionsvorrichtung also als Nutzerschnittstelle aufgefasst werden, die dem Nutzer eine haptische Eingabe von Nutzereingaben ermöglicht.

Dabei kann die Interaktionsvorrichtung ein oder mehrere berührungssensitive Eingabeelemente bzw. Eingabefelder umfassen ("Touch-Bedienelemente"), über die der Nutzer Nutzereingaben eingeben kann. Die Eingabeelemente werden im Speziellen als Bedienelemente bezeichnet. Beispielsweise umfasst die Interaktionsvorrichtung als Eingabeelemente und/oder Eingabefelder einen Ziffernblock, einen Buchstabenblock, Pfeilfelder (up, down, right, left) und/oder Eingabefelder mit festgelegter oder festlegbarer Funktion.

Eine wenigstens teilweise Überdeckung einer Fläche des Glaselements kann beispielsweise bedeuten, dass die Interaktionsvorrichtung die Fläche zu wenigstens 20%, bevorzugt zu wenigstens 40%, insbesondere bevorzugt zu wenigstens 60% und weiter bevorzugt zu wenigstens 80% oder aber auch vollständig überdeckt. Die Fläche kann dabei einer Fläche des Glaselements gegenüberliegen, mit der das Glaselement zu einer eine ionisierende Strahlung emittierenden Quelle auszurichten ist. Die Fläche und damit das Glaselement können dabei so dimensioniert sein, dass ein Nutzer wirksam vor ionisierender Strahlung abgeschirmt werden kann. Z.B. kann die Fläche eine Fläche von wenigstens 0,5 m², bevorzugt von wenigstens 1 m² und weiter bevorzugt von wenigstens 1,5 m² aufweisen. Das Glaselement kann eine Dicke von 4 - 25 mm, bevorzugt 4 - 10 mm, aufweisen.

Die Interaktionsvorrichtung kann dabei in einem Sichtbereich des Nutzers durch das Glaselement angeordnet sein, wenn der Nutzer durch das Glaselement blickt.

Durch die wenigstens teilweise Überdeckung des Glaselements mit der Interaktionsvorrichtung ermöglicht die Strahlenschutzvorrichtung einem Nutzer, eine strahlensichere Position hinter der Strahlenschutzvorrichtung einzunehmen und sich gleichzeitig Informationen anzeigen zu lassen und/oder über entsprechende Bedienelemente Nutzereingaben, insbesondere Steuerbefehle, einzugeben. Dadurch wird eine verbesserte Kontrolle von Prozessen unter Verwendung von ionisierender Strahlung aussendenden Geräten durch einen Nutzer gewährleistet. Gleichzeitig kann ein ausreichender Strahlungsschutz des Nutzers sichergestellt werden.

Die Strahlenschutzvorrichtung umfasst eine Freigabevorrichtung. Die Freigabevorrichtung ist ausgebildet, eine Freigabeeingabe durch einen Nutzer zu empfangen. Die Freigabevorrichtung ist insbesondere flächig ausgebildet. Insbesondere ist die Freigabevorrichtung wenigstens abschnittsweise transparent oder transluzent ausgebildet. Die Freigabevorrichtung überdeckt vorzugsweise wenigstens einen Teil einer Fläche des Glaselements.

Beispielsweise umfasst oder bildet die Freigabevorrichtung eine berührungsempfindliche Schicht, insbesondere eine transparente und/oder transluzente berührungsempfindliche Schicht. Optional ist die Freigabevorrichtung flächig ausgebildet zum Darstellen von Daten auf einer Fläche. Insbesondere kann die Freigabevorrichtung also als Nutzerschnittstelle aufgefasst werden, die dem Nutzer eine haptische Eingabe der Freigabeeingabe ermöglicht. Die Freigabeeingabe ist insbesondere eine Eingabe zur Freigabe der Nutzereingabe und/oder zur Bestätigung der Nutzereingabe. Insbesondere ist die Freigabeeingabe nötig, um die Nutzereingabe zu bestätigen, insbesondere um die Nutzereingabe zur Ausführung, Weitergabe und/oder Steuerung zu bringen.

Dabei kann die Freigabevorrichtung ein oder mehrere berührungssensitive Eingabeelemente bzw. Eingabefelder umfassen ("Touch-Bedienelemente"), über die der Nutzer Freigabeeingaben eingeben kann. Die Eingabeelemente werden im Speziellen als Bedienelemente und/oder Freigabeelement bezeichnet.

Eine wenigstens teilweise Überdeckung einer Fläche des Glaselements kann beispielsweise bedeuten, dass die Freigabevorrichtung die Fläche zu wenigstens 20%, bevorzugt zu wenigstens 40%, insbesondere bevorzugt zu wenigstens 60% und weiter bevorzugt zu wenigstens 80% oder aber auch vollständig überdeckt. Die Fläche kann dabei zur Quelle der ionisierenden Strahlung ausgerichtet sein oder einer Fläche des Glaselements gegenüberliegen, mit der das Glaselement zu einer eine ionisierende Strahlung emittierenden Quelle auszurichten ist.

Die Freigabevorrichtung kann dabei in einem Sichtbereich des Nutzers durch das Glaselement angeordnet sein, wenn der Nutzer durch das Glaselement blickt. Alternativ ist die Freigabevorrichtung außerhalb des obigen Sichtbereichs angeordnet.

Durch die wenigstens teilweise Überdeckung des Glaselements mit der Interaktionsvorrichtung und/oder der Freigabevorrichtung ermöglicht die Strahlenschutzvorrichtung einem Nutzer, eine strahlensichere Position hinter der Strahlenschutzvorrichtung einzunehmen und sich gleichzeitig Informationen anzeigen zu lassen und/oder über entsprechende Bedienelemente Nutzereingaben, insbesondere Steuerbefehle einzugeben. Durch die Verwendung der Interaktionsvorrichtung und der Freigabevorrichtung können ein unbeabsichtigtes Bedienen, ein unbeabsichtigtes Ausführen einer Nutzereingabe und/oder ungewollte Steuerung vermieden werden. Dadurch wird eine verbesserte Kontrolle von Prozessen und sicherere Steuerung und/oder Bedienung von Modalitäten, die ionisierende Strahlung aussendenden, gewährleistet. Gleichzeitig kann ein ausreichender Strahlungsschutz des Nutzers sichergestellt werden.

Insbesondere umfassend eine Schnittstelle und ein Steuermodul. Die Schnittstelle und das Steuermodul sind insbesondere datentechnisch verbunden. Die Schnittstelle ist zur Ausgabe eines Steuersignals ausgebildet. Die Schnittstelle bildet insbesondere eine Datenschnittstelle. Die Schnittstelle kann eine Kabelschnittstelle oder eine kabellose (wireless) Schnittstelle bilden, beispielsweise eine Funk-, WLAN- oder Bluetooth-Schnittstelle. Die Schnittstelle ist vorzugsweise zur Kopplung und/oder Verbindung mit der medizinischen und/oder bildgebenden Modalität und/oder mit einem Bewegungsautomat, wobei der Bewegungsautomat beispielsweise zur Bewegung, Steuerung oder Regelung eines Patiententisches, Manipulators und/oder eines medizinischen Gerätes ausgebildet ist. Dem Steuermodul ist die empfangene Nutzereingabe bereitgestellt. Vorzugsweise ist dem Steuermodul der Zeitpunkt oder ein Zeitstempel des Empfangens der Nutzereingabe von der Interaktionsvorrichtung bereitgestellt. Dem Steuermodul ist die empfangene Freigabeeingabe bereitgestellt. Vorzugsweise ist dem Steuermodul der Zeitpunkt oder ein Zeitstempel des Empfangens der Freigabeeingabe von der Freigabevorrichtung bereitgestellt. Das Steuermodul ist ausgebildet, das Steuersignal an der Schnittstelle auszugeben, wenn die Nutzereingabe und die Freigabeeingabe gleichzeitig oder innerhalb eines zeitlichen Sicherheitsfensters erfolgen und/oder empfangen werden. Mit anderen Worten ist das Steuermodul ausgebildet, zu prüfen, ob die Nutzereingabe und die Freigabeeingabe gleichzeitig und/oder zeitlich innerhalb des Sicherheitsfensters erfolgten und/oder empfangen wurden, wobei das Steuermodul das Steuersignal bestimmt und/oder ausgibt, wenn Freigabe- und Nutzereingabe gleichzeitig bzw. innerhalb des Sicherheitsfensters erfolgten oder empfangen wurden. Insbesondere wird von dem Steuermodul kein Steuersignal bestimmt und/oder an der Schnittstelle ausgegeben, wenn Freigabe- und Nutzereingabe nicht gleichzeitig oder innerhalb des Zeitfensters erfolgten. Im Speziellen ist das Steuermodul ausgebildet, die Ausgabe eines Steuersignals an der Schnittstelle zu unterbinden und/oder zu stoppen, wenn Freigabe- und Nutzereingabe nicht gleichzeitig oder innerhalb des Zeitfensters erfolgten. Das Steuermodul kann ein Hardwaremodul und/oder Softwaremodul bilden und/oder umfassen.

Die Interaktionsvorrichtung umfasst eine erste Sensorfolie als Interaktionsschnittstelle für den Nutzer, wobei die erste Sensorfolie zur Eingabe und/oder zum Empfangen der Nutzereingabe durch den Nutzer ausgebildet ist. Die erste Sensorfolie ist insbesondere flexibel und/oder biegeschlaff. Die erste Sensorfolie ist insbesondere flächig ausgebildet und/oder weist eine Dicke bzw. Folienstärke von weniger als 10 mm, vorzugsweise weniger als 5 mm und im Speziellen von weniger als 2 mm auf. Die erste Sensorfolie kann insbesondere auf elektroaktiven Polymeren basieren, elektrische und/oder elektronische Komponenten, Leiterbahnen und/oder Schichten umfassen.

Die Freigabevorrichtung umfasst insbesondere eine zweite Sensorfolie als Interaktionsschnittstelle für den Nutzer, wobei die zweite Sensorfolie zur Freigabe der Nutzereingabe und/oder zum Empfangen der Freigabeeingabe durch den Nutzer ausgebildet ist. Die zweite Sensorfolie ist insbesondere flexibel und/oder biegeschlaff. Die zweite Sensorfolie ist insbesondere flächig ausgebildet und/oder weist eine Dicke bzw. Folienstärke von weniger als 10 mm, vorzugsweise weniger als 5 mm und im Speziellen von weniger als 2 mm auf. Die zweite Sensorfolie kann insbesondere auf elektroaktiven Polymeren basieren, elektrische und/oder elektronische Komponenten, Leiterbahnen und/oder Schichten umfassen.

Vorzugsweise umfasst die Interaktionsvorrichtung und/oder die Freigabevorrichtung eine Kraftmessfolie. Im Speziellen bildet die erste Sensorfolie und/oder die zweite Sensorfolie die Kraftmessfolie. Die Kraftmessfolie ist insbesondere auf einer Fläche, insbesondere dem Glaselement, angebracht und/oder anbringbar. Die Kraftmessfolie ist zur Erfassung einer auf die Kraftmessfolie einwirkenden Kontaktkraft ausgebildet. Die Kraftmessfolie umfasst eine Anbringschicht, mit der die Kraftmessfolie auf der Fläche, insbesondere dem Glaselement, angebracht ist. Die Anbringschicht umfasst beispielsweise einen Klebstoff und/oder ist zur stoffschlüssigen Verbindung mit dem Glaselement bzw. der Fläche ausgebildet. Beispielsweise ist die Kraftmessfolie auf der Fläche, bzw. dem Glaselement aufgeklebt und/oder laminiert. Die Kraftmessfolie bildet insbesondere eine berührungssensitive Folie und/oder Schicht.

Die Kraftmessfolie umfasst insbesondere eine Sensorschicht, die derart ausgebildet ist, dass sich ein elektrischer Widerstand der Sensorschicht als Funktion der (von der Deckschicht an die Sensorschicht übertragenen) Kontaktkraft ändert und wenigstens ein auf dem elektrischen Widerstand basierendes Messsignal abgegriffen werden kann.

Als Kontaktkraft kann eine auf die Kraftmessfolie bzw. auf eine an einer Fläche des Glaselements angebrachte Kraftmessfolie einwirkende mechanische Belastung oder Kraft verstanden werden, insbesondere eine von dem Nutzer durch Berührung mit den Fingern bewirkte und/oder beaufschlagende Kraft. Die Kontaktkraft kann beispielsweise durch eine händische Eingabe, Fingereingabe (Touch-Eingabe) oder ein Objekt (Eingabestift) vermittelt werden, wobei eine mechanische Kraft oder Belastung auf die Kraftmessfolie ausgeübt wird.

Die Kraftmessfolie ist dazu ausgelegt, eine auf sie und damit auf die Interaktionsvorrichtung und/oder Freigabevorrichtung einwirkende Kontaktkraft zu erfassen. Insbesondere ist die Kraftmessfolie die Nutzereingabe und/oder Freigabeeingabe zu erfassen. Insbesondere bildet eine von der Kraftmessfolie erfasste Nutzereingabe bzw. Freigabeeingabe die empfangene Nutzereingabe bzw. Freigabeeingabe. Die Sensorschicht ist derart ausgebildet, dass eine auf die Kraftmessfolie einwirkende Kontaktkraft durch einen in Abhängigkeit der einwirkenden Kontaktkraft veränderlichen elektrischen Widerstand erfassbar ist. Mit anderen Worten kann die Sensorschicht als ein piezo-resistives Kraftmesselement bezeichnet werden. Ferner kann die Kraftmessfolie als piezo-resistive Kraftmessfolie bezeichnet werden. Insbesondere kann die Sensorschicht zwischen der Deckschicht und der Abdeckschicht angeordnet sein.

Zur Bereitstellung solcher piezo-resistiven Eigenschaften kann die Sensorschicht ein piezo-resistives Material oder ein oder mehrere piezo-resistive Elemente aufweisen, die einzeln oder in Kombination eine piezo-resistiven Eigenschaft der Kraftmessfolie bereitstellen.

"Als Funktion" oder "in Abhängigkeit" der Kontaktkraft veränderlich kann dabei insbesondere heißen, dass die Kraftmessfolie bzw. die Sensorschicht wenigstens zwei verschiedene elektrische Widerstandswerte bei unterschiedlichen Kraftwerten der einwirkenden Kontaktkraft bereitstellt. In Ausführungsformen kann der elektrische Widerstand der Kraftmessfolie bzw. der Sensorschicht wenigstens abschnittsweise mit steigender Kontaktkraft zu- oder abnehmen. Insbesondere kann der elektrische Widerstand der Kraftmessfolie bzw. der Sensorschicht wenigstens abschnittsweise proportional zu einer steigenden Kontaktkraft zu- oder abnehmen. Ferner kann die Sensorschicht derart ausgebildet sein, dass ein Schwellenwert hinsichtlich der einwirkenden Kontaktkraft überwunden werden muss, oberhalb dessen sich der elektrische Widerstand der Kraftmessfolie bzw. der Sensorschicht ändert.

Der elektrische Widerstand der Kraftmessfolie bzw. der Sensorschicht wird mittels einem oder mehrerer Messwerte erfasst, die von der Kraftmessfolie bzw. der Sensorschicht abgegriffen werden können. Die Messwerte können beispielsweise elektrische Ströme sein. Zur Ermöglichung des Abgreifens der Messwerte kann die Kraftmessfolie bzw. die Sensorschicht ein oder mehrere elektrische Anschlüsse aufweisen, die Teil einer Schnittstelle der Kraftmessfolie sein können oder mit einer solchen in Verbindung stehen können.

Die Kraftmessfolie und damit die darin enthaltenen Schichten können wenigstens abschnittsweise flexibel sein. Flexibel kann insbesondere heißen, dass die Kraftmessfolie und die darin enthaltenen Schichten bis zu einem gewissen Grad in ein oder mehrere Richtungen biegbar sind ohne Schaden zu nehmen. Beispielsweise kann die Kraftmessfolie eine Dicke von 50 µm bis zu 10 cm aufweisen. Bevorzugt weist die Kraftmessfolie eine Dicke von 1 mm bis 20 mm auf.

Die Deckschicht kann allgemein die nach außen, d.h. von dem Bewegungselement bzw. der Anbringschicht weg, gerichtete Oberfläche oder Schicht der Kraftmessfolie bezeichnen. Die Deckschicht kann Teil der Sensorschicht sein oder separat von dieser ausgebildet sein. Die Deckschicht kann dazu ausgebildet sein, die Kraftmessfolie und insbesondere die Sensorschicht vor Umwelteinflüssen zu schützen und/oder die Kraftmessfolie durch eine Hinterdruckung oder Bedruckung kenntlich zu machen. Die Deckschicht kann beispielsweise aus einem Kunststoffmaterial ausgebildet sein oder ein solches umfassen. Insbesondere kann die Deckschicht aus einem Polymermaterial ausgebildet sein oder ein solches umfassen.

Die Anbringschicht kann allgemein eine Oberfläche oder Schicht der Kraftmessfolie bezeichnen, mit der die Kraftmessfolie an eine Fläche angebracht werden kann. Die Anbringschicht kann zum Anbringen der Kraftmessfolie auf eine Fläche ausgebildet sein. D.h. die Anbringschicht kann dazu ausgebildet sein, eine Kontaktfläche oder Anbringfläche zu dieser Fläche bereitzustellen. Die Anbringschicht kann Teil der Sensorschicht sein oder separat von dieser ausgebildet sein. Die Anbringschicht kann ein oder mehrere Anbringelemente zum Anbringen der Kraftmessfolie auf eine Fläche aufweisen. Beispielsweise können diese Anbringelemente Ausnehmungen umfassen, in die entsprechende vorstehende Befestigungselemente der Fläche eingreifen können oder die Anbringelemente können vorstehende Befestigungselemente umfassen, die in entsprechende Ausnehmungen in der Fläche eingreifen können. Insbesondere kann die Anbringschicht wenigstens abschnittsweise ein adhäsives Material zum Aufkleben der Kraftmessfolie auf die Fläche aufweisen. Die Anbringschicht kann beispielsweise aus einem Kunststoffmaterial ausgebildet sein oder ein solches umfassen. Insbesondere kann die Anbringschicht aus einem Polymermaterial ausgebildet sein oder ein solches umfassen. Insbesondere ist dem Steuermodul das abgegriffene Messignal, insbesondere das von der Kontaktkraft abhängige Messignal, bereitgestellt. Das Steuermodul kann ausgebildet sein ein von dem Messsignal und/oder von der Kontaktkraft abhängiges Steuersignal zu bestimmen und/oder auszugeben. Mit anderen Worten kann das Steuersignal funktional abhängig, insbesondere proportional, zur Kontaktkraft und/oder dem Messsignal ausgebildet sein. Beispielsweise kann eine stärkere Kontaktkraft bei der Eingabe der Benutzereingabe ein größeres oder dazu proportionales Steuersignal bewirken, beispielsweise eine schnellere Bewegung der gesteuerten Patientenliege.

Besonders bevorzugt weist die Interaktionsvorrichtung ein berührungssensitives Eingabeelement zum Empfangen der Nutzereingabe durch den Nutzer auf und/oder weist die Freigabevorrichtung ein berührungssensitives Eingabeelement zum Empfangen der Freigabeeingabe durch den Nutzer auf. Das berührungssensitive Eingabeelement implementiert eine Berührungserkennung, die ausgewählt ist aus:
- einer optischen Berührungserkennung,
- einer kapazitiven Berührungserkennung,
- einer induktiven Berührungserkennung,
- einer resistiven Berührungserkennung,
- einer Infrarot-basierten Berührungserkennung, die insbesondere als in-Glas Berührungserkennung ausgeführt ist, und/oder
- Kombinationen der vorgenannten Berührungserkennungen.

Eine optische Berührungserkennung kann dabei beispielsweise eine Kameravorrichtung, insbesondere eine Stereo-Kameravorrichtung oder ToF-Sensoren (Time of Flight), umfassen, mit der ein Berührpunkt des Nutzers mit der Interaktionsvorrichtung und/oder Freigabevorrichtung erfasst werden kann.

Ferner kann eine optische Berührungserkennung einen Array (eine Anordnung) Licht-emittierender Elemente und einen Array (eine Anordnung) korrespondierender Sensorelemente aufweisen, welche Sensorelemente dazu ausgebildet sind, Licht der Licht-emittierenden Elemente zu erfassen. Die Licht-emittierenden Elemente können z.B. LEDs sein. Die Sensorelemente können z.B. Photodetektoren sein. Insbesondere ist das Array Licht-emittierender Elemente dem Array korrespondierender Sensorelemente über das Glaselement, die Freigabevorrichtung und/oder die Interaktionsvorrichtung hinweg gegenüberliegend angeordnet. Insbesondere weisen das Licht-emittierende Array und das Array korrespondierender Sensorelemente jeweils zwei Abschnitte auf, die senkrecht zueinander angeordnet sind. Insbesondere können die Licht-emittierenden Elemente dazu ausgebildet sein, eine Infrarotstrahlung zu emittieren. Insbesondere können die Licht-emittierenden Elemente und die Sensorelemente jeweils an einer Kante des Glaselements, also seitlich am Glaselement angeordnet sein, sodass die Lichtausbreitung von den Licht-emittierenden Elementen im Wesentlichen im Glaselement verläuft. Insbesondere können die Licht-emittierenden Elemente derart ausgebildet sein, dass sie kollimiertes Licht aussenden. Eine Anordnung kann z.B. jeweils eine Mehrzahl an Licht-emittierenden Elementen und korrespondierenden Sensorelementen umfassen, etwa mehr als 20 oder mehr als 40 oder mehr als 80 pro Seite des Glaselements. Berührt ein Nutzer das Glaselement, wird das von den Licht-emittierenden Elementen emittierte Licht abgelenkt, was in einem veränderten Signal der Sensorelemente messbar wird.

Mit anderen Worten kann so eine Infrarot-basierte Berührungserkennung bereitgestellt werden. Dies kann beispielsweise ein Array von infraroten LED und Photodetektor-Paaren umfassen, die gegenüberliegend entlang wenigstens zweier Kanten des Glaselements oder eines separaten Abdeckelements angeordnet sind. Es ergibt sich ein Muster an horizontalen und vertikalen LED-Strahlen. Eine Berührung führt zu lokalen Strahlungsverlusten und damit zu einer Veränderung des Musters, welche durch die Photodetektoren messbar wird. Die LEDs und Photodetektoren können dabei derart an den Kanten des Glaselements angeordnet sein, dass die LEDs in das Glaselement abstrahlen und die Photodetektoren die aus dem Glas austretende Strahlung messen. Eine solche Anordnung wird "in-Glas Berührungserkennung" oder Berührungserkennung mit seitlicher Beleuchtung genannt.

Eine kapazitive Berührungserkennung kann beispielsweise mittels einer durchsichtigen Metalloxid-Beschichtung (Metalloxid-Funktionsschicht) realisiert werden. Eine an den Ecken der Beschichtung angelegte Wechselspannung erzeugt ein konstantes, gleichmäßiges elektrisches Feld. Bei Berührung entsteht ein geringer Ladungstransport, der im Entladezyklus in Form eines Stromes an den Ecken gemessen wird. Die resultierenden Ströme aus den Ecken stehen im direkten Verhältnis zu der Berührungsposition. Die Metalloxidschicht kann z.B. direkt auf das Glaselement oder ein separates Abdeckelement aufgebracht bzw. auflaminiert werden.

Eine andere Bauart für kapazitive Berührungserkennung (meistens "PCT" = "Projected Capacitive Touch" genannt) nutzt zwei Funktionsschichten mit einem leitfähigen Muster (meistens Streifen oder Rauten). Die Funktionsschichten sind voneinander isoliert angebracht und bilden damit lokal Kondensatorelemente aus. Eine Funktionsschicht dient als Sensor, die andere übernimmt die Aufgabe des Treibers. Befindet sich z.B. ein Finger des Nutzers auf der Sensor-Funktionsschicht, so ändert sich die Kapazität des Kondensatorelements, und es kommt ein größeres Signal an der Treiber-Funktionsschicht an. Die Sensor-Funktionsschicht kann auf einem Abdeckelement angebracht sein, das parallel zum Glaselement verläuft (insbesondere in einer Sandwich-Anordnung), während die Treiber-Funktionsschicht auf dem Glaselement angeordnet ist.

Eine resistive Berührungserkennung reagiert auf Druck, der zwei elektrisch leitfähige Funktionsschichten stellenweise verbindet. Die Funktionsschichten bilden einen Spannungsteiler, an dem der elektrische Widerstand gemessen wird, um die Position der Druckstelle zu ermitteln.

Eine induktive Berührungserkennung kann durch eine Metallnetz-Funktionsschicht hinter einem parallel zum Glaselement angeordneten Abdeckelement realisiert sein, das ein Magnetfeld aufbaut. Berührt ein spezielles Bedienelement (z.B. ein Bedienstift) das Abdeckelement, erzeugt eine Spule im Stift einen Strom. Dieser Strom wird als Signal von horizontal und vertikal ausgerichteten Antennen der Interaktionsvorrichtung empfangen.

Durch die genannten Systeme zur Berührungserkennung kann eine zuverlässige Eingabe von Nutzereingaben und/oder Freigabeeingaben gewährleistet werden. Gleichzeitig sind die genannten Systeme gut mit der Strahlenschutzvorrichtung kombinierbar, da z.B. das Glaselement in den Aufbau der Berührungserkennung einbezogen werden kann. Ferner können z.B. Multitouch-Events wie Zoom-In/Zoom-Out/Rotate bzw. Increase/Decrease für Sliderfunktionen unterstützt werden.

Insbesondere kann die Interaktionsvorrichtung verschiedene Touch- oder Slider-Elemente aufweisen. Diese können z.B. durch die vorgenannten berührungssensitiven Eingabeelemente bereitgestellt werden.

Gemäß einem Aspekt wird eine Strahlenschutzvorrichtung bereitgestellt, bei der die Interaktionsvorrichtung ein Abdeckelement und ein Funktionselement umfasst. Das Abdeckelement ist parallel zum Glaselement (parallel zu der Fläche des Glaselements) angeordnet. Das Funktionselement ist zwischen Abdeckelement und Glaselement angeordnet und zum Darstellen von Daten für den Nutzer und/oder zum Empfangen einer Nutzereingabe durch den Nutzer ausgebildet.

Vorzugsweise ist die Interaktionsvorrichtung auf dem Glaselement angeordnet, insbesondere in einem Eingabeabschnitt des Glaselements. Die Freigabevorrichtung kann auf dem Glaselement angeordnet sein, in einem Freigabeabschnitt des Glaselements. Der Freigabeabschnitt und/oder der Eingabeabschnitt sind insbesondere flächige Abschnitte und/oder Bereiche des Glaselements. Der Eingabeabschnitt und der Freigabeabschnitt sind beabstandet angeordnet. Insbesondere sind so beabstandet angeordnet, dass Freigabeabschnitt und Eingabeabschnitt nicht gleichzeitig mit einer Hand berührt werden können. Beispielsweise sind der Eingabeabschnitt und der Freigabeabschnitt auf zwei gegenüberliegenden Flächen oder gegenüberliegenden Seiten einer Fläche angeordnet. Insbesondere sind Freigabeabschnitt und Eingabeabschnitt mindestens 1 cm, optional mindestens 5 cm oder 10 cm, vorzugsweise mindestens 20 cm und im Speziellen mindestens 50 cm voneinander beabstandet.

Das Glaselement umfasst insbesondere eine flächige Frontseite und eine flächige Rückseite. Die Frontseite ist dabei beispielsweise die Seite des Glaselements, die dem Nutzer zugwendet ist und/oder von der medizinischen, bildgebenden und/oder Strahlung emittierenden Modalität abgewendet ist. Die Rückseite liegt der Frontseite gegenüber und/oder die Rückseite ist die Seite des Glaselements, die von dem Nutzer abgewendet ist und/oder der medizinischen, bildgebenden und/oder Strahlung emittierenden Modalität zugewendet ist. Der Eingabeabschnitt ist vorzugsweise auf der Frontseite angeordnet, wobei der Freigabeabschnitt auf der Rückseite angeordnet ist. Alternativ ist der Eingabeabschnitt auf der Rückseite angeordnet, wobei der Freigabeabschnitt auf der Frontseite angeordnet ist. Ferner kann in einer möglichen Ausgestaltung der Eingabeabschnitt und der Freigabeabschnitt auf der gleichen flächigen Seite des Glaselements angeordnet sein, vorzugsweise auf der Frontseite.

Besonders bevorzugt ist der Eingabeabschnitt und/oder der Freigabeabschnitt in einem Randbereich des Glaselements angeordnet. Als Randbereich wird insbesondere ein Bereich des Glaselements, insbesondere der Frontseite und/oder der Rückseite verstanden, der an den Rändern, Enden und/oder Kanten angeordnet ist. Beispielsweise kann als ein Randbereich ein flächiger Bereich der Frontseite verstanden werden, der sich von den Kanten bzw. Rändern der flächigen Frontseite ein Randabstandsmaß, insbesondere senkrecht zum Rand bzw. der Kante, erstreckt. Mit anderen Worten ist der Randbereich beispielsweise rahmenförmig ausgebildet. Beispielsweise ist das Glaselement und/oder die Frontseite rechteckig, wobei der Randbereich einen rechteckigen Rahmen bildet. Der Eingabeabschnitt und der Freigabeabschnitt sind vorzugsweise gegenüberliegenden Randbereichen der Frontseite oder Rückseite angeordnet. Beispielsweise ist der Freigabeabschnitt in einem aus Nutzersicht rechten Randbereich angeordnet, wobei der Eingabeabschnitt in einem aus Nutzersicht linken Randbereich angeordnet ist. Im Speziellen kann es vorgesehen sein, dass Eingabeabschnitt und Freigabeabschnitt auf unterschiedlichen Seiten (Rückseite/Vorderseite) angeordnet, wobei der Eingabeabschnitt und der Freigabeabschnitt hierbei im gleichen Randbereich angeordnet sind, sodass der Nutzer den Eingabeabschnitt und Freigabeabschnitt mit einer Hand durch Umgreifen des Glaselements am Rand bzw. an den Kanten erfassen kann. Beispielsweise ist der Eingabeabschnitt in einem Randbereich auf der Vorderseite angeordnet sind, z.B. rechts, wobei auf der Rückseite in einem Randbereich hinter dem Eingabeabschnitt der Freigabeabschnitt angeordnet ist.

Vorzugsweise umfasst die Interaktionsvorrichtung ein oder mehrere vorgefertigte Bedienfelder zur Eingabe einer Nutzereingabe. Gemäß einem Aspekt weist die Interaktionsvorrichtung ein oder mehrere vorgefertigte Bedienfelder jeweils zur Eingabe einer Nutzereingabe auf. Im Speziellen umfasst die Freigabevorrichtung ein oder mehrere vorgefertigte Bedienfelder zur Eingabe der Freigabeeingabe.

Die Bedienfelder können beispielsweise in das Glaselement und/oder das Abdeckelement eingearbeitet oder auf das Glaselement und/oder das Abdeckelement aufgebracht sein. Beispielsweise können die Bedienfelder in das Glaselement und/oder das Abdeckelement eingeätzt, eingebrannt oder eingelasert oder auf diese aufgemalt oder aufgeklebt sein. Mit anderen Worten können in der Interaktionsvorrichtung und/oder Freigabevorrichtung permanente Bedienfelder vorgesehen sein, die insbesondere wiederkehrende Steuerbefehle des Nutzers betreffen (z.B. Start, Stopp, Pause).

Gemäß einem Aspekt können die Bedienfelder ferner beleuchtet sein, insbesondere durch eine Anzeigevorrichtung. Das Beleuchten der Bedienfelder kann insbesondere einen ersten Anzeigezustand bilden, wobei ein zweiter Anzeigezustand oder weitere Anzeigezustand durch Beleuchten der Bedienfelder in einer anderen Farbe, blinkendes Beleuchten oder deaktivieren der Beleuchtung gebildet wird. Das Beleuchten der Bedienfelder, insbesondere durch die Anzeigevorrichtung kann beispielsweise durch eine seitliche Beleuchtung des Glaselements und/oder Abdeckelements mit sichtbarem Licht erfolgen. Dabei kann das seitlich eingestrahlte Licht durch den Glaskörper/Abdeckelement-Körper geleitet werden und sich in den Bedienfeldern brechen und diese dadurch beleuchten. Durch die Bereitstellung von insbesondere beleuchteten Bedienfeldern wird dem Nutzer die Eingabe von Nutzereingaben erleichtert.

Optional umfasst die Strahlenschutzvorrichtung eine Anzeigevorrichtung zur Anzeige des Erhaltens der Nutzereingabe, des Erhaltens der Freigabeeingabe und/oder der Ausgabe des Steuersignals. Insbesondere ist die Anzeigevorrichtung ausgebildet eine optische Anzeige, Licht- und/oder Leuchtsignal auszugeben, wenn die Nutzereingabe und die Freigabeeingabe gleichzeitig vorliegen bzw. erfolgten und/oder wenn die Nutzereingabe und die Freigabeeingabe innerhalb des Sicherheitsfensters erfolgten und/oder vorliegen. Alternativ und/oder ergänzend ist die Anzeigevorrichtung das Empfangen der Nutzereingabe, insbesondere für die Dauer des Sicherheitsfensters anzuzeigen. Dadurch wird dem Nutzer angezeigt, wie lange der Nutzer zur Freigabe der Nutzereingabe Zeit hat.

Als Anzeigen des Empfangens der Nutzereingabe wird insbesondere verstanden, dass angezeigt wird, dass die Nutzereingabe prinzipiell erkannt und/oder erfasst wurde, wobei dies vorzugsweise unabhängig davon ist, ob die Nutzereingabe verarbeitbar und/oder interpretierbar ist. Als Anzeigen des Empfangens der Freigabeeingabe wird insbesondere verstanden, dass angezeigt wird, dass die Freigabeeingabe prinzipiell erkannt und/oder erfasst wurde, wobei dies vorzugsweise unabhängig davon ist, ob die Freigabeeingabe verarbeitbar und/oder interpretierbar ist. Als Anzeigen der Ausgabe des Steuersignals wird insbesondere verstanden, dass Angezeigt wird, dass das Steuermodul das Steuersignal bestimmt hat und/oder an der Schnittstelle ausgibt. Mit anderen Worten wird durch das Anzeigen des Ausgebens des Steuersignals angezeigt, dass die Nutzereingabe durch die Freigabeeingabe freigegeben wurde und/oder dass Nutzereingabe und Freigabeeingabe zeitgleich bzw. innerhalb des Sicherheitsfensters erfolgten. Im Speziellen ist die Anzeigevorrichtung ausgebildet, das Erhalten unterschiedliche Nutzereingaben unterschiedlich anzuzeigen, insbesondere unterschiedlich optisch anzuzeigen. Das optische Anzeigen ist vorzugsweise eine temporäre Anzeige, beispielsweise für ein Anzeigeintervall auch Anzeigedauer genannt, wobei das Anzeigeintervall vorzugsweise kleiner als 2 Sekunden ist. Mit anderen Worten ist die Anzeigevorrichtung ausgebildet, das Empfangen der Nutzereingabe durch Ausgabe einer optischen Anzeige, eines optischen Signals und/oder eines Licht- und/oder Leuchtsignals zu bestätigen und/oder dem Benutzer eine Rückmeldung zu geben, dass die Nutzereingabe empfangen wurde und der Benutzer gegebenenfalls weitere Nutzereingaben vornehmen kann bzw. mit der Nutzereingabe fortfahren kann.

Als optisches Anzeigen wird insbesondere ein Ausgeben eines optischen Signals, beispielsweise Licht- oder Leuchtsignals verstanden, beispielsweise ein Aufleuchten oder Aktivieren einer Lichtquelle. Das optische Anzeigen und/oder das optische Signal kann monochrom oder mehrfarbig, zeitlich konstant oder zeitlich variierend ausgebildet sein, beispielsweise zeitlich variierend in einer Intensität oder Farbe. Im Speziellen kann die Anzeigevorrichtung ausgebildet sein, das Empfangen der Nutzereingabe, der Freigabeeingabe und/oder des Ausgebens des Steuersignal zusätzlich zur optischen Anzeige akustisch anzuzeigen und/oder zu melden. Im Speziellen ist die Anzeigevorrichtung ausgebildet, das Empfangen der Nutzereingabe, das Empfangen der Freigabeeingabe und/oder das Ausgeben des Steuersignals verschieden anzuzeigen, beispielsweise durch verschiedene Anzeigezustände.

Die Anzeigevorrichtung ist insbesondere mit dem Glaselement verbunden. Beispielsweise ist die Anzeigevorrichtung flächig ausgebildet und auf einer Vorderseite oder Rückseite des Glaselements angebracht, vorzugsweise auf das Glaselement laminiert oder stoffschlüssig mit dem Glaselement verbunden. Ferner kann die Anzeigevorrichtung die Interaktionsvorrichtung, die Freigabevorrichtung und/oder das Glaselement mindestens abschnittsweise und/oder in eine Umlaufrichtung umgeben. Beispielsweise ist die Anzeigevorrichtung an Seitenflächen und/ oder Kanten des Glaselements, der Interaktionsvorrichtung und/oder Freigabevorrichtung angeordnet, insbesondere zum Einkoppeln von elektromagnetischer Strahlung, des Licht- oder Leuchtsignals.

Das optische Anzeigen der Anzeigevorrichtung kann beispielsweise durch Einkoppeln des Licht- und/oder Leuchtsignals in das Glaselement, in die Interaktionsvorrichtung, die Freigabevorrichtung und/oder ein Abdeckelement ausgebildet sein. Alternativ und/oder ergänzend umfasst die Anzeigevorrichtung eine lokale, insbesondere punktförmige oder flächige, Lichtquelle, z.B. eine LED, wobei die Anzeigevorrichtung ausgebildet ist, das Leucht- und/oder Lichtsignal mittels der Lichtquelle, insbesondere lokal, punktförmig oder flächig, auszugeben.

Beispielsweise ist die Anzeigevorrichtung ausgebildet, zum optischen Anzeigen, das transparente und/oder transluzente Glaselement, Abdeckelement, die Freigabevorrichtung oder Interaktionsvorrichtung temporär, insbesondere für die Anzeigedauer, zu beleuchten bzw. das Licht- und/oder Leuchtsignal einzukoppeln. Im Speziellen umfasst das Glaselement, das Abdeckelement, Freigabevorrichtung oder die Interaktionsvorrichtung Lichtstreubereiche, beispielsweise durch eingebracht Materialveränderungen, wobei die Lichtstreubereich ausgebildet sind, eingekoppelte Licht- oder Leuchtsignale zu streuen bzw. emittieren. Das Anzeigen wird von der Anzeigevorrichtung beispielsweise durch Aktivieren und/oder Deaktivieren der Leucht- bzw. Lichtquelle realisiert.

Beispielsweise ist die Anzeigevorrichtung, die Interaktionsvorrichtung, Freigabevorrichtung und/oder das Glaselement im normalen Betrieb unbeleuchtet, wobei normaler Betrieb hierbei als Betrieb ohne Vorliegen einer Benutzereingabe oder Freigabeeingabe verstanden wird. Zum optischen Anzeigen durch Anzeigevorrichtung, kann die Anzeigevorrichtung, die Interaktionsvorrichtung, die Freigabevorrichtung und/oder das Glaselement beleuchtet werden, wobei das Beleuchten vorzugsweise für die Anzeigedauer erfolgt. Alternativ ist die Anzeigevorrichtung, die Freigabevorrichtung, die Interaktionsvorrichtung und/oder das Glaselement im normalen Betrieb beleuchtet, wobei das Beleuchten in einer ersten Farbe erfolgt, wobei zum optischen Anzeigen durch Anzeigevorrichtung, die Anzeigevorrichtung, die Freigabevorrichtung, die Interaktionsvorrichtung und/oder das Glaselement in einer zweiten Farbe beleuchtet wird oder die Beleuchtung deaktiviert wird, wobei das Beleuchten in der zweiten Farbe oder das Deaktivieren der Beleuchtung vorzugsweise für die Anzeigedauer erfolgt.

Diese Ausgestaltung ermöglicht es einem Nutzer geschützt von der ionisierenden Strahlung mit Blick auf eine bildgebende Modalität diese zu steuern, wobei der Nutzer sofortiges Feedback über seine Nutzereingabe und/oder Freigabeeingabe erhält.

Besonders bevorzugt ist es, dass die Anzeigevorrichtung mindestens zwei optische Anzeigezustände aufweist und/oder einnehmen kann. Insbesondere ist die Anzeigevorrichtung ausgebildet, zwischen den mindestens zwei optischen Anzeigezuständen zu wechseln. Die unterschiedlichen optischen Anzeigezustände sind von einem menschlichen Auge als unterschiedliche optische Zustände erkenntlich, beispielsweise anhand unterschiedlicher Farbe, Intensität, Beleuchtung, Ausgabebereich oder anderweitiger vom menschlichen Auge erfassbare Parameter des Leucht- oder Lichtsignals. Die optischen Anzeigezustände werden auch als Beleuchtungszustände bezeichnet, sodass mit anderen Worten die Anzeigevorrichtung ausgebildet ist, mindestens zwei verschiedene Beleuchtungszustände anzuzeigen und/oder einzunehmen. Die Anzeigevorrichtung ist hierbei ausgebildet, das Anzeigen durch einen mindestens temporären Wechsel zwischen mindestens zwei Anzeigezuständen zu realisieren. Beispielsweise weist die Anzeigevorrichtung zwei Anzeigezustände auf, einen ersten und einen zweiten Anzeigezustand, wobei die Anzeigevorrichtung durch einen Wechsel vom ersten in den zweiten Anzeigezustand das Empfangen der Nutzereingabe, der Freigabeeingabe und/oder das Ausgeben des Steuersignals anzeigt.

Im Speziellen ist es vorgesehen, dass die Anzeigevorrichtung zur Ausgabe eines elektromagnetischen Signals und/oder zur Ausgabe elektromagnetischer Strahlung ausgebildet ist, insbesondere wird durch die Ausgabe der elektromagnetischen Strahlung optisch angezeigt, dass die Nutzereingabe oder Freigabeeingabe empfangen wurde. Die elektromagnetische Strahlung ist insbesondere eine elektromagnetische Strahlung im Wellenlängenbereich zwischen 400 und 800 nm. Mit anderen Worten ist die ausgegebene elektromagnetische Strahlung eine vom menschlichen Auge erfassbare Strahlung. Im Speziellen bildet das elektromagnetische Signal das Leucht- oder Lichtsignal. Insbesondere ist es vorgesehen, dass sich die mindestens zwei optischen Anzeigezustände in mindestens einem, die elektromagnetische Strahlung charakterisierten Parameter, unterscheiden. Beispielsweise unterscheiden sich die optischen Anzeigezustände durch die Ausgabe von elektromagnetischer Strahlung unterschiedlicher Intensität, unterschiedlicher spektraler Zusammensetzung, unterschiedlicher Wellenlänge, unterschiedlicher Strahlungsleistung oder bei Ausgabe eines Blinksignals in einer Blinkfrequenz. Insbesondere kann einer der optischen Anzeigezustände durch die Ausgabe von elektromagnetischer Strahlung der Strahlungsleistung und/oder Intensität gleich Null ausgebildet sein. Mit anderen Worten kann einer der optischen Anzeigezustände als ein Deaktivieren und/oder nicht betreiben einer Leucht- und/oder Lichtquelle gebildet sein.

Besonders bevorzugt ist es, dass die Anzeigevorrichtung ausgebildet ist, das Empfangen der Benutzereingabe durch einen Wechsel zwischen den mindestens zwei Anzeigezuständen bzw. zwischen den exakt zwei Anzeigezuständen anzuzeigen. Besonders bevorzugt ist der Wechsel vorübergehend und/oder temporär, sodass beispielsweise vom ersten in den zweiten Anzeigezustand gewechselt wird und nach einer Anzeigedauer zurück in den ersten Anzeigezustand gewechselt wird. Die Anzeigedauer beträgt vorzugsweise weniger als 10 Sekunden, im Speziellen weniger als 5 Sekunden und besonders bevorzugt weniger als 1 Sekunde. Durch die Begrenzung der Anzeigedauer kann die Eingabe einer Mehrzahl von Benutzereingaben besonders schnell erfolgen, da der Nutzer für die nächste Benutzereingabe und dessen Empfangsanzeige nicht übermäßig warten muss.

Optional ist es vorgesehen, dass die Interaktionsvorrichtung ausgebildet ist, unterschiedliche Nutzereingaben zu verarbeiten und/oder zu erhalten. Insbesondere kann als die Nutzereingabe eine aus einer Mehrzahl an auswählbaren Benutzereingaben ausgewählte Benutzereingaben bilden. Besonders bevorzugt ist, dass die Anzeigevorrichtung ausgebildet ist, das Empfangen von unterschiedlichen Nutzereingaben unterschiedlich optisch anzuzeigen, beispielsweise durch unterschiedliche Anzeigezustände. Besonders bevorzugt weist die Anzeigevorrichtung die gleiche Anzahl optischer Anzeigezustände auf, wie von der Interaktionsvorrichtung auswählbare Benutzereingaben, sodass jedem der unterschiedlichen möglichen Benutzereingaben ein charakteristischer Anzeigezustand zugeordnet ist.

Eine Ausgestaltung der Erfindung sieht vor, dass die Anzeigevorrichtung eine Folienanzeige oder ein Folienleuchtmittel umfasst. Als Folienanzeige wird insbesondere eine Anzeige verstanden, welche eine Dicke von weniger als 5 mm aufweist. Im Speziellen wird als Folienanzeige oder Folienleuchtmittel eine Anzeige und/oder ein Leuchtmittel mit flächiger Erstreckung verstanden, welches insbesondere biegeschlaff ausgebildet ist. Besonders bevorzugt bildet die Folienanzeige, dass Folienleuchtmittel und/oder die Anzeigevorrichtung eine OLED (organic light-emitting diode). Die Folienanzeige und/oder das Folienleuchtmittel ist insbesondere auf dem Glaselement befestigt, beispielsweise angeklebt, laminiert oder anderweitig form-, kraft- oder stoffschlüssig verbunden. Im Speziellen kann die Anzeigevorrichtung, bzw. die Folienanzeige oder das Folienleuchtmittel zwischen der Interaktionsvorrichtung und dem Glaselement angeordnet sein, beispielsweise zum hinterleuchten und/oder optischen Anzeigen in der Interaktionsvorrichtung.

Gemäß einem Aspekt wird eine Strahlenschutzvorrichtung bereitgestellt, bei der die Interaktionsvorrichtung ein Abdeckelement und ein Funktionselement umfasst. Das Abdeckelement ist parallel zum Glaselement (parallel zu der Fläche des Glaselements) angeordnet. Das Funktionselement ist zwischen Abdeckelement und Glaselement angeordnet und zum Darstellen von Daten für den Nutzer und/oder zum Empfangen einer Nutzereingabe durch den Nutzer ausgebildet.

Das Abdeckelement ist insbesondere transparent. Das Abdeckelement kann als Kunststoffscheibe (z.B. aus Polycarbonat) oder zweites Glaselement ausgebildet sein. Das zweite Glaselement kann als Displayglas oder Abdeckglas ausgebildet sein. Gemäß einigen Beispielen ist das zweite Glaselement nicht als Schutzglas gegen ionisierende Strahlung ausgebildet und verfügt z.B. in der Glaszusammensetzung über einen Bleigehalt von weniger als 60 Gew.-%.

Insbesondere ist das Abdeckelement derart an dem Glaselement angeordnet oder angebracht, dass zwischen Glaselement und Abdeckelement ein o.g. Funktionselement angeordnet ist, welches zum Darstellen von Daten und/oder zum Empfangen einer Nutzereingabe ausgebildet ist (ggf. in Wechselwirkung mit weiteren Komponenten der Interaktionsvorrichtung). Das Funktionselement kann eine oder mehrere der o.g. Berührungserkennungen implementieren und damit ein oder mehrere der o.g. berührungssensitiven Eingabeelemente umfassen. Das Funktionselement kann eine oder mehrere Funktionsschichten aufweisen, beispielsweise die Metalloxid-Funktionsschicht einer kapazitiven Berührungserkennung, die PCT-Funktionsschichten, die elektrisch leitfähigen Funktionsschichten der resistiven Berührungserkennung, die LCD-, TFT-, OLED-Funktionsschichten und/oder die Metallnetz-Funktionsschicht der induktiven Berührungserkennung.

Gemäß einem Aspekt kann das Abdeckelement selbst zum Darstellen der Daten und/oder zum Empfangen der Nutzereingabe ausgebildet sein. Dazu kann die Interaktionsvorrichtung derart ausgebildet sein, dass das Abdeckelement seitlich beleuchtet wird, etwa zur Realisierung einer in-Glas Berührungserkennung mit den oben beschriebenen Arrays aus Licht-emittierenden Elementen und Sensorelementen.

Optional weist das Abdeckelement eine flächige Elementvorderseite und eine flächige Elementrückseite. Beispielsweise ist auf der Elementvorderseite die Interaktionsvorrichtung angeordnet und/oder Bedienelemente der Interaktionsvorrichtung sind auf der Elementvorderseite angeordnet, wobei auf der Elementrückseite die Freigabevorrichtung und/oder Bedienelemente der Freigabevorrichtung angeordnet sind.

Die Erfindung betrifft ferner eine bildgebende Modalität mit einer Strahlenschutzvorrichtung nach einem der vorgenannten Aspekte. Die bildgebende Modalität umfasst und/oder bildet ein Röntgengerät, einen C-Bogen, einen Computertomographen oder ein Bestrahlungsgerät für eine Strahlentherapie.

Weitere Besonderheiten und Vorteile der Erfindung werden aus den nachfolgenden Erläuterungen von Ausführungsbeispielen anhand von schematischen Zeichnungen ersichtlich. In diesem Zusammenhang genannte Modifikationen können jeweils miteinander kombiniert werden, um neue Ausführungsformen auszubilden. In unterschiedlichen Figuren werden für gleiche Merkmale die gleichen Bezugszeichen verwendet.
- Figur 1: zeigt eine schematische Darstellung eines Systems zur bildgebenden Untersuchung eines Patienten unter Verwendung einer eine ionisierende Strahlung aussendenden Modalität.
- Figur 2: zeigt eine Strahlenschutzvorrichtung gemäß einer Ausführungsform.
- Figur 3: zeigt eine Strahlenschutzvorrichtung gemäß einer weiteren Ausführungsform.
- Figuren 4a und 4b: zeigen einen Ausschnitt einer Strahlenschutzvorrichtung gemäß einer Ausführungsform.
- Figur 5: zeigt eine Interaktionsvorrichtung gemäß einer Ausführungsform im Detail.
- Figur 6: zeigt eine Interaktionsvorrichtung gemäß einer weiteren Ausführungsform im Detail.

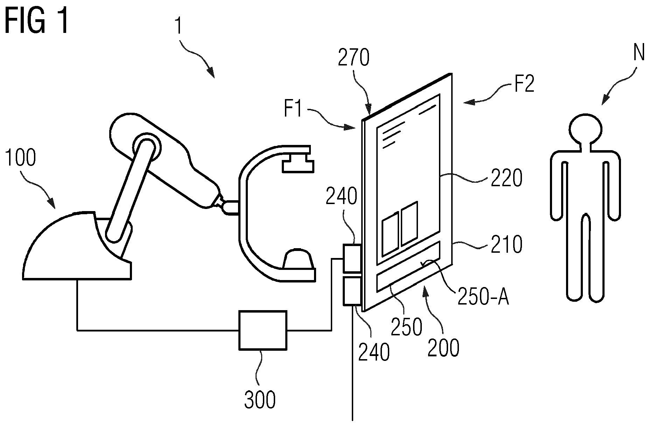

In Figur 1 ist ein System 1 zur Durchführung einer bildgebenden Untersuchung eines Patienten dargestellt. Die bildgebende Untersuchung kann z.B. während eines interventionellen Eingriffs am Patienten erfolgen. Die bildgebende Untersuchung kann dabei durch einen Nutzer N kontrolliert und/oder gesteuert werden. Das System 1 weist eine bildgebende Modalität 100 und eine Strahlenschutzvorrichtung 200 auf. Das System 1 kann ferner noch weitere, nicht gezeigte Vorrichtungen aufweisen, wie etwa eine Patientenliege, eine Interventionsvorrichtung zur Durchführung eines interventionellen Eingriffs am Patienten oder weitere Einrichtungen.

Die bildgebende Modalität 100 kann insbesondere eine Modalität sein, die auf einer Erzeugung von Röntgenstrahlen mittels wenigstens einer Röntgenquelle beruht. Beispielsweise kann die bildgebende Modalität als Röntgengerät, wie etwa als C-Bogen-Röntgengerät oder als Computertomografiegerät ausgebildet sein.

Zur Abschirmung des Nutzers N vor gestreuter Röntgenstrahlung weist das System 1 ferner eine Strahlenschutzvorrichtung 200 auf. Die Strahlenschutzvorrichtung 200 weist ein transparentes bzw. für den Nutzer durchsichtiges Glaselement 210 auf, das als Schutzglas gegen Röntgenstrahlung ausgebildet ist. Das Glaselement 210 kann beispielsweise als Bleiglas oder Bleiacrylglas ausgebildet sein und in der Glaszusammensetzung einen Bleianteil von wenigstens 60 Gewichtsprozent (Gew.-%) aufweisen. Bezüglich des Glaselements 210 lassen sich zwei Seiten bzw. Flächen definieren. Eine zur ionisierenden Strahlung auszurichtende Rückseite oder erste Fläche F1 und eine dieser ersten Fläche F1 gegenüberliegende zweite Fläche F2 oder auch Rückseite genannt. Zum Schutz vor ionisierender Strahlung hält sich der Nutzer N auf der zweiten Seite F2 auf. Das Glaselement 210 kann dabei derartige Abmessungen aufweisen, dass der Nutzer N hinter dem Glaselement 210 gut vor ionisierender Strahlung geschützt ist. Beispielsweise kann das Glaselement 210 rechteckig ausgebildet sein und z.B. eine Höhe von 1,5 m und eine Breite von 1 m aufweisen. Andere Abmessungen und Formen sind selbstverständlich möglich. Insbesondere kann das Glaselement 210 auch gewölbt sein. Insbesondere kann das Glaselement 210 derart gewölbt sein, dass es auf der zweiten Seite F2 konkav ausgebildet ist.

Die Strahlenschutzvorrichtung 200 weist ferner einer Interaktionsvorrichtung 220 auf, mit der der Nutzer N mit dem System 1 in Wechselwirkung treten kann. Die Interaktionsvorrichtung 220 ist zum Empfang von Nutzereingaben ausgebildet. Insbesondere kann die Interaktionsvorrichtung 220 zur Anzeige von Daten ausgebildet sein. Die Nutzereingaben werden dabei bevorzugt über Touch-Bedienung in die entsprechend ausgebildete Interaktionsvorrichtung 220 eingegeben. Die Interaktionsvorrichtung 220 ist auf der Vorderseite des Glaselements bzw. der Fläche F2 angeordnet.

Die Strahlenschutzvorrichtung 200 weist ferner einer Freigabevorrichtung 270 auf, mit der der Nutzer N mit dem System 1 in Wechselwirkung treten kann. Die Freigabevorrichtung 270 ist zum Empfang von Freigabeeingaben ausgebildet. Insbesondere kann die Freigabevorrichtung 270 zur Anzeige von Daten ausgebildet sein. Die Freigabeeingaben werden dabei bevorzugt über Touch-Bedienung in die entsprechend ausgebildete Freigabevorrichtung 270 eingegeben. Die Freigabevorrichtung 270 ist auf der Rückseite des Glaselements 210 bzw. der Fläche F1 angeordnet.

Wie in Figur 1 gezeigt, ist die Interaktionsvorrichtung 220 so in der Strahlenschutzvorrichtung 200 angeordnet, dass sich die Interaktionsvorrichtung 220 im Sichtbereich eines hinter der Strahlenschutzvorrichtung 200 stehenden Nutzers N befindet oder leicht in den Sichtbereich des Nutzers N gebracht werden kann. Die Interaktionsvorrichtung 220 überdeckt wenigstens einen Teil des Glaselements 210. Bevorzugt ist die Interaktionsvorrichtung 220 auf der zweiten Seite F2 des Glaselements 210 angeordnet.

Die Freigabevorrichtung 270 ist vorzugsweise in einer Draufsicht auf die Interaktionsvorrichtung aus Nutzersicht auf der Rückseite angeordnet. Insbesondere weißen Interaktionsvorrichtung 220 und Freigabevorrichtung 270 in einer Projektion senkrecht zur Fläche F1 oder F2 eine Überlappung auf. Die Interaktionsvorrichtung 220 und die Freigabevorrichtung 270 sind wenigstens abschnittsweise transparent ausgebildet, sodass der Nutzer N durch die Interaktionsvorrichtung 220, die Freigabevorrichtung 270 und das Glaselement 210 in den hinter der Strahlenschutzvorrichtung 200 liegenden Raum blicken kann.

Die Strahlenschutzvorrichtung 200 weist ferner eine Anzeigevorrichtung 250 auf, mit der dem Nutzer N das Empfangen einer Nutzereingabe oder einer Freigabeeingabe angezeigt wird. Die Anzeigevorrichtung 250 ist ausgebildet, eine mittels der Interaktionsvorrichtung 220 vorgenommene Nutzereingabe des Nutzers N anzuzeigen, bzw. anzuzeigen, dass die Nutzereingabe von der Interaktionsvorrichtung 220 empfangen wurde. Die Anzeigevorrichtung 250 kann ferner ausgebildet, eine mittels der Freigabevorrichtung vorgenommene Freigabeeingabe des Nutzers N anzuzeigen, bzw. anzuzeigen, dass die Freigabeeingabe von der Freigabevorrichtung 270 empfangen wurde. Die Anzeigevorrichtung 250 ist ausgebildet, optisch anzuzeigen, beispielsweise durch Aussenden eines Leucht- oder Lichtsignals, dass die Nutzereingabe und/oder Freigabeeingabe empfangen wurde.

Wie in Figur 1 gezeigt, ist die Anzeigevorrichtung 250 so in der Strahlenschutzvorrichtung 200 angeordnet, dass sich die Anzeige im Sichtbereich eines hinter der Strahlenschutzvorrichtung 200 stehenden Nutzers N befindet oder leicht in den Sichtbereich des Nutzers N gebracht werden kann. Beispielsweise umfasst die Anzeigevorrichtung 250 einen flächigen Abschnitt 250-A, der auf der Seite F2 oder F1, des Glaselements 210 angeordnet ist und zur Anzeige aufleuchtet und/oder das Licht- oder Leuchtsignal aussendet. Die Anzeigevorrichtung 250 überdeckt wenigstens einen Teil des Glaselements 210. Die Anzeigevorrichtung 250 ist optional wenigstens abschnittsweise transparent ausgebildet, sodass der Nutzer N durch die Interaktionsvorrichtung 220 und das Glaselement 210 in den hinter der Strahlenschutzvorrichtung 200 liegenden Raum blicken kann.

Zur Weitergabe von Nutzereingaben und/oder zum Empfang von Daten zur Anzeige weist die Interaktionsvorrichtung 220 ferner eine (Daten-)Schnittstelle 240 auf. Zur Versorgung der mit elektrischer Energie weist die Strahlenschutzvorrichtung 220 ferner eine Stromversorgungsvorrichtung auf, welche mit der Schnittstelle 240 ein gemeinsames Modul bilden kann. Die Schnittstelle 240 kann beispielsweise mit einer Recheneinheit 300 in Verbindung stehen. Von der Recheneinheit 300 kann die Interaktionsvorrichtung 220 über die Schnittstelle 240 z.B. Daten zur Anzeige empfangen. Ferner können Nutzereingaben über die Schnittstelle 240 an die Recheneinheit 300 übermittelt werden. Die Recheneinheit 300 kann beispielsweise als Recheneinheit 300 des Systems 1 bzw. der bildgebenden Modalität implementiert sein.

Ferner kann die Strahlenschutzvorrichtung 200 ein integriertes Steuermodul 260 aufweisen. Das Steuermodul 260 ist ausgebildet, zu prüfen, ob eine empfangene Nutzereingabe freigegeben wurde. Insbesondere ist das Steuermodul 260 ausgebildet, nur freigegebene Nutzereingaben zur Steuerung weiterzugeben und/oder zur Ausführung zuzulassen. Das Steuermodul 270 prüft, ob zur empfangenen Nutzereingabe gleichzeitig oder innerhalb eines zeitlichen Sicherheitsfenster eine Freigabeeingabe empfangen wird oder empfangen wurde. Für den Fall, dass zu einer Nutzereingabe gleichzeitig oder innerhalb des Sicherheitsfensters eine Freigabeeingabe empfangen wurde, bestimmt das Steuermodul 260 ein Steuersignal. Das von dem Steuermodul 260 bestimmte Steuersignal wird an der Schnittstelle 240 ausgegeben. Insbesondere ist die Schnittstelle 240 mit der Modalität 100 oder der Recheneinheit 300 verbunden. Wobei die Recheneinheit 300 oder die Modalität 100 zur Ausführung der Nutzereingabe bzw. dem Steuersignal ausgebildet ist. Die Recheneinheit 300 und die integrierte Steuereinheit 260 können zusammen Teil einer Recheneinrichtung des Systems 1 sein.

Die Strahlenschutzvorrichtung 200 kann ferner eine Stativvorrichtung 230-DS, 230-RS aufweisen, um die Strahlenschutzvorrichtung 200 relativ zur bildgebenden Modalität 100 und dem Nutzer zu positionieren.

Die mit der Interaktionsvorrichtung 220 anzeigbaren Daten können z.B. Statusdaten der bildgebenden Modalität 100 umfassen. Ferner können Daten angezeigt werden, welche die mit der bildgebenden Modalität 100 durchzuführende Untersuchung betreffen, wie etwa Patientendaten, medizinische Bilddaten oder Umgebungsdaten. Die Nutzereingaben können sich auf die Steuerung der bildgebenden Modalität 100 oder peripherer Funktionen in Zusammenhang mit der durchzuführenden Untersuchung beziehen. Beispielsweise kann per Nutzereingabe in die Interaktionsvorrichtung 220 eine Bildaufnahme mit der bildgebenden Modalität 100 gestartet oder gestoppt werden, Bildgebungsparameter können eingestellt werden, Daten können angefordert werden oder Umgebungsparameter des Untersuchungsraums, wie etwa eine Beleuchtung, können eingestellt werden.

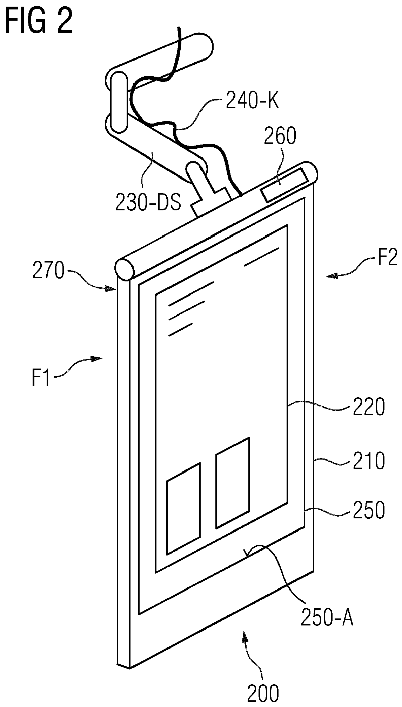

In Figur 2 ist eine Ausführungsform der Strahlenschutzvorrichtung 200 in größerem Detail gezeigt. Die Anzeigevorrichtung umfasst den flächigen Abschnitt 250-A zur optischen Anzeige und/oder der Ausgabe des Leucht- oder Lichtsignals. Der flächige Abschnitt 250-A ist umlaufend um die Interaktionsvorrichtung 220 angeordnet. Mit anderen Worten bildet die Anzeigevorrichtung 250 und/oder der flächige Abschnitt 250-A einen beleuchtbaren Ring um die Interaktionsvorrichtung 220.

In der gezeigten Ausführungsform ist die Strahlenschutzvorrichtung 200 in deckenmontierter Ausführung dargestellt. Entsprechend ist die Stativvorrichtung als Deckenstativ 230-DS ausgeführt. Durch die Verwendung einer Deckenstativvorrichtung 230-DS können sowohl die Stromversorgung als auch die Datenverbindung kabelgebunden ausgeführt werden, ohne dass die entsprechenden Kabel den Nutzer oder Freiheitsgrade in der Positionierung der Strahlenschutzvorrichtung 200 einschränken. Die Schnittstelle 240 kann entsprechend als kabelgebundene Schnittstelle 240-K ausgeführt sein. In ähnlicher Weise kann die Stromversorgungvorrichtung als kabelgebundene Stromversorgungsvorrichtung ausgebildet sein.

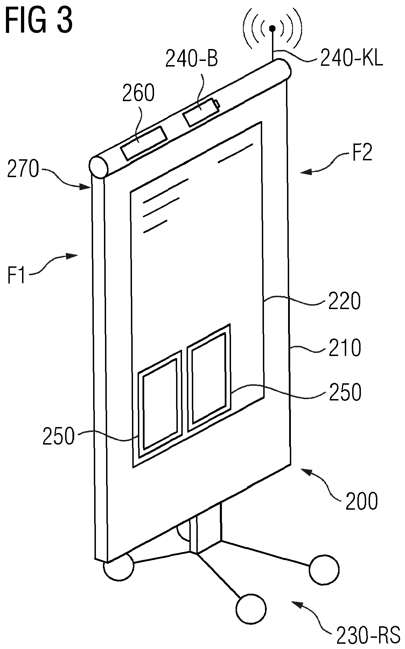

In einer in Figur 3 dargestellten alternativen Ausführungsform ist die Strahlenschutzvorrichtung 200 als rollbare Strahlenschutzvorrichtung 200 ausgeführt. Entsprechend ist die Stativvorrichtung als Boden-Rollstativ 230-RS ausgebildet. Um eine möglichst freie Positionierbarkeit der Strahlenschutzvorrichtung 200 zu gewährleisten ist die Schnittstelle 240 als kabellose Schnittstelle 240-KL ausgebildet. Eine solche kabellose Schnittstelle 240-KL kann beispielsweise als WLAN-, Bluetooth- oder ZigBee-Schnittstelle implementiert sein. Die Stromversorgungsvorrichtung weist in dieser Ausführungsform bevorzugt eine Batterie bzw. einen Akkumulator auf.

Die Darstellung der Schnittstellen 240 bzw. Stromversorgungsvorrichtungen ist dabei lediglich beispielhaft und nicht einschränkend zu verstehen. So kann auch die Decken-montierte Ausführungsform von Figur 2 anstelle der kabelgebundenen Schnittelle 240-K oder zusätzlich dazu mit einer kabellosen Schnittstelle 240-KL ausgestattet sein und anstelle der kabelgebundenen Stromversorgungsvorrichtung oder zusätzlich dazu eine Batterie aufweisen. Umgekehrt kann selbstverständlich auch die die rollbare Strahlenschutzvorrichtung 200 von Figur 3 eine kabelgebundene Schnittstelle 240-K bzw. Stromversorgungsvorrichtung aufweisen.

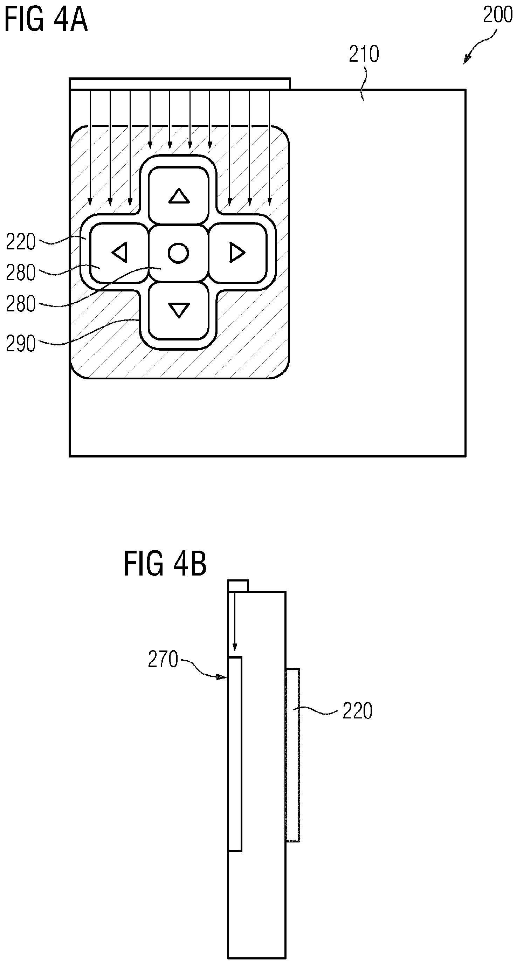

In Figur 4a ist ein Ausschnitt einer Strahlenschutzvorrichtung 200 in einer Draufsicht aus Nutzersicht gezeigt. Auf der Vorderseite des Glaselements 210 ist eine Interaktionsvorrichtung 220 angeordnet. Die Interaktionsvorrichtung 220 weist Bedienelemente, auch Bedienfelder 280, auf. Die Bedienfelder 280 umfassen hierbei Pfeiltasten zur Steuerung der Bewegung einer Vorrichtung, beispielsweise eines Manipulators, einer Robotoerkineamtik, eines Aktors oder eines Patiententisches. Das Glaselement 210 und/oder die Interaktionsvorrichtung 220 umfasst eine Gravur 290, die zur Hinterleuchtung der Interaktionsvorrichtung 220, im Speziellen der Bedienfelder, und/oder der Freigabevorrichtung 270 ausgebildet ist. An einer Kante des Glaselements 210 ist eine Licht- und/oder Leuchtquelle 250-BE angeordnet. Die Licht- und/oder Leuchtquelle 250-BE ist Teil der Anzeigevorrichtung 250. Die Lichtund/oder Leuchtquellen sind ausgebildet elektromagnetische Strahlung, als ein Licht- und/oder Leuchtsignal bzw. als Licht, in das Glaselement 210 einzukoppeln, wobei das eingekoppelte Licht in der Gravur 290 gestreut wird und für den Nutzer N als Hinterleuchtung aufgefasst wird. Durch das Glaselement 210 und insbesondere durch die transparente Interaktionsvorrichtung 220 ist für den Nutzer die auf der Rückseite angeordnete Freigabevorrichtung 270 sichtbar, zumindest schemenhaft. Die Freigabevorrichtung 270 und die Interaktionsvorrichtung 220 sind aus der Sicht bzw. einer hinter der Strahlenschutzvorrichtung 200 angeordneten Nutzer N mit Blick auf die Modalität 100, beide am linken Rand, bzw. linken Randbereich, des Glaselements 210 angeordnet. Der Nutzer N kann so mit einer Hand, bzw. zwei Fingern, insbesondere Daumen und Zeigefinger, gleichzeitig eine Nutzereingabe an der Interaktionsvorrichtung 220 und eine Freigabeeingabe an der Freigabevorrichtung 270 tätigen. Das Glaselement 210 ist vorzugsweise aus PMMA.

Figur 4b zeigt den Ausschnitt der Strahlenschutzvorrichtung 200 aus Figur 4a in einer Seitenansicht, insbesondere auf die aus Nutzersicht linke Kante des Glaselements 210. Insbesondere ist aus der Seitenansicht erkenntlich, dass sowohl die Interaktionsvorrichtung 220 als auch die Freigabevorrichtung 270 flach ausgebildet sind, insbesondere als Folien auf dem Glaselement 210 angeordnet sind. Die Anzeigevorrichtung 250, insbesondere die Leuchtquelle 250-BE, ist ausgebildet eine das Empfangen einer Freigabeeingabe anzuzeigen, wobei hierbei die Gravur 290 nahe oder im Bereich der Freigabevorrichtung 270 angeordnet werden kann, sodass die eingekoppelte elektromagnetische Strahlung in der Gravur 290 gestreut wird und als Hinterleuchtung der Freigabevorrichtung 270 aufgefasst wird.

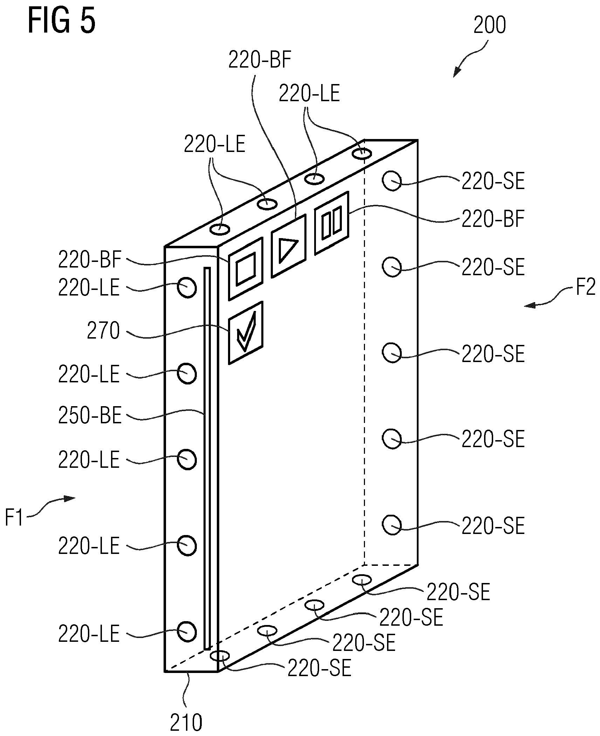

In **Figur 5** ist eine Interaktionsvorrichtung 220 gemäß einer Ausführungsform im Detail dargestellt. In der gezeigten Ausführungsform ist das Glaselement 210 integraler Bestandteil der Interaktionsvorrichtung 220.

Daneben können in dem Glaselement 210 vorbestimmte Bedienfelder 220-BF angeordnet bzw. eingearbeitet sein. Mit anderen Worten sind die Bedienfelder physisch in oder auf dem Glaselement 210 angeordnet. Die vorbestimmten Bedienfelder 220-BF können beispielsweise durch eine geeignete Bearbeitung in das Glaselement 210 eingearbeitet sein, etwa durch Ätzen oder Laser-Bearbeitung. Mittels einer seitlichen Beleuchtung durch wenigstens eine entsprechend an den Kanten des Glaselements 210 angeordnete Anzeigevorrichtung 250 können die Bedienfelder 220-BF hervorgehoben werden, indem sich durch die Anzeigevorrichtung eingestrahltes sichtbares Licht in den Bedienfeldern 220-BF bricht bzw. gestreut wird. Die vorbestimmten Bedienfelder 220-BF können z.B. auf einige Standard-Nutzereingeben gerichtet sein, wie Start, Pause oder Stopp der bildgebenden Aufnahme. Die Anzeigevorrichtung 250 ist ausgebildet, zum Anzeigen des Empfangens der Nutzereingabe unterschiedlich Licht einzustrahlen. Beispielsweise wird zum Anzeigen des Empfangs der Nutzereingabe das Einstrahlen von sichtbarem Licht und/oder das Hervorheben der Bedienfelder für eine Anzeigedauer unterbrochen oder farblich verändert.

Eine Berührungserkennung kann in dieser Ausführungsform durch eine Infrarot-Sensorik realisiert werden. Zu diesem Zweck können an wenigstens einer Kante des Glaselements 210 Infrarot-Licht emittierende Leuchtelement 220-LE angeordnet sein und an einer gegenüberliegenden Kante können entsprechende Sensorelement 220-SE angeordnet sein, die eine Lichtintensität bzw. Lichtmenge des von den Leuchtelementen 220-LE ausgesandten Infrarot-Lichts messen. Die Leuchtelemente 220-LE können z.B. als Leuchtdioden ausgeführt sein, während die Sensorelemente 220-SE durch Photodetektoren, etwa in Form von Fotodioden, gebildet werden. Bevorzugt sind sowohl Leuchtelemente 220-LE als auch Sensorelemente 220-SE an gegenüberliegenden Kanten des Glaselements 210 in x- und y-Richtung angeordnet, sodass sich ein Gitter aus Infrarot-Strahlen in dem Glaselement 210 ergibt. Erfolgt eine Berührung des Glaselements 210, wird ein Teil des Lichts eines entsprechenden Strahls gebeugt bzw. gestreut, da sich die Reflexionseigenschaften an der Grenzfläche ändern. Diese Veränderung wird als Signal in den Sensorelementen 220-SE messbar und ein zugehöriger Berührpunkt kann bestimmt werden.

Als Alternative kann eine Berührungserkennung über eine optische Berührungserkennung erfolgen. Dazu kann ein Kamerasystem (nicht dargestellt) and einer Seite des Glaselements 210 angeordnet werden und eine Berührung durch den Nutzer registrieren. Bevorzugt ist ein solches Kamerasystem als Stereo-Kamerasystem mit zwei Kameras ausgebildet, um den Berührpunkt durch Aufnahme aus verschiedenen Richtungen bestimmen zu können.

In Figur 5 ist eine weitere Ausführungsform der Interaktionsvorrichtung 200 dargestellt. Die Interaktionsvorrichtung 200 weist ein wenigstens teilweise transparentes Abdeckelement 220-AB auf, das parallel zu dem Glaselement 210 an der zweiten Seite F2 der Strahlenschutzvorrichtung 200 angeordnet ist. Das Abdeckelement 220-AB kann beispielsweise aus Glas oder Kunststoff ausgebildet sein. Im Gegensatz zum Glaselement 210 weist das Abdeckelement 220-AB in dieser Ausführungsform keine Schutzeigenschaften gegen ionisierende Strahlung auf (eine Ausführung des Abdeckelements 220-AB weiteres Strahlenschutzelement z.B. als Strahlenschutzglas ist aber natürlich nicht ausgeschlossen). Insbesondere kann das Abdeckelement 220-AB als Displayglas ausgebildet sein. Das Abdeckelement 220-AB kann im Wesentlichen die gleiche flächige Form wie das Glaselement 210, also die gleiche Höhe, Breite oder Wölbung aufweisen.

Das Abdeckelement 220-AB und das Glaselement 210 definieren in dieser Ausführungsform zwischen sich einen Zwischenraum ZR. In den Zwischenraum ZR ist ein Funktionselement 220-FE der Interaktionsvorrichtung 220 angeordnet. Mit anderen Worten ist das Funktionselement 220-FE von dem Glaselement 210 und dem Abdeckelement 220-AB "gesandwicht". Das Funktionselement 220-FE ist derart ausgebildet, dass es, ggf. in Zusammenwirkung mit dem Glaselement 210 und/oder dem Abdeckelement 220-AB, eine oder mehrere Funktionen zur Anzeige von Daten für den Nutzer N und/oder zum Empfangen einer Nutzereingabe realisiert. Dazu kann das Funktionselement 220-FE eine oder mehrere Funktionsschichten 220-FS aufweisen. Die Anzeigevorrichtung 250 ist angeordnet und/oder ausgebildet, Licht in das Abdeckelement 220-AB oder die Interaktionsvorrichtung 220 einzukoppeln, sodass das optische Anzeigen in oder mittels des Abdeckelements 220-AB erfolgt. Gleichsam können ein oder mehrere Beleuchtungselemente 250-BE von der Anzeigevorrichtung 250 zur seitlichen Beleuchtung des Abdeckelements 220-AB umfasst sein.

In einer Ausführungsform kann eine Funktionsschicht 220-FE beispielsweise eine LCD-, OLED- oder TFT-Schicht umfassen, mit der Daten durch das Abdeckelement 220-AB angezeigt werden können.

Zur Realisierung einer kapazitiven Berührungserkennung kann eine Funktionsschicht beispielsweise mittels einer durchsichtigen Metalloxid-Beschichtung realisiert werden. Eine an den Ecken der Beschichtung angelegte Wechselspannung erzeugt ein konstantes, gleichmäßiges elektrisches Feld. Bei Berührung entsteht ein geringer Ladungstransport, der im Entladezyklus in Form eines Stromes an den Ecken gemessen wird. Die resultierenden Ströme aus den Ecken stehen im direkten Verhältnis zu der Berührungsposition. Die Metalloxidschicht kann als Funktionsschicht 220-FS z.B. direkt auf das Glaselement 210 oder das Abdeckelement 220-AB aufgebracht werden.

Eine andere Bauart für kapazitive Berührungserkennung (meistens "PCT" = "Projected Capacitive Touch") nutzt zwei Funktionsschichten 220-FS jeweils mit einem leitfähigen Muster (z.B. Streifen oder Rauten). Diese Funktionsschichten 220-FS sind voneinander isoliert angebracht, z.B. durch eine weitere Funktionsschicht 220-FS. Eine Funktionsschicht 220-FS dient als Sensor, die andere übernimmt die Aufgabe des Treibers. Befindet sich z.B. ein Finger des Nutzers auf einer Funktionsschicht 220-FS, so ändert sich die Kapazität der durch die Funktionsschichten 220-FS gebildeten Kondensatoranordnung, und es kommt ein größeres Signal an der Empfänger-Funktionsschicht 220-FS an. Die Sensor-Funktionsschicht 220-FS kann auf dem Abdeckelement 220-AB angebracht sein, während die Treiber-Funktionsschicht 220-FS auf dem Glaselement 210 angeordnet ist.

In dem Funktionselement 220-FE kann ferner eine resistive Berührungserkennung realisiert sein. Dazu kann das Funktionselement 220-FE zwei voneinander getrennte, elektrisch leitfähige Funktionsschichten 220-FS aufweisen. Die beiden Funktionsschichten 220-FS können z.B. durch eine Abstandshalter-Funktionsschicht 220-FS voneinander getrennt sein. Die Funktionsschichten 220-FS bilden so einen Spannungsteiler. Bei Druckausübung auf die Anordnung verändert sich der elektrische Widerstand, der zwischen den elektrisch leitfähigen Funktionsschichten 220-FS abfällt.

Ferner kann das Funktionselement 220-FE eine induktive Berührungserkennung aufweisen. Dazu kann im Zwischenraum ZR eine Funktionsschicht 220-FS als ein Metallnetz realisiert sein, das ein Magnetfeld aufbaut. Berührt ein spezielles Bedienelement (z.B. ein Bedienstift) das Abdeckelement 220-AB, erzeugt eine Spule im Stift einen Strom. Dieser Strom wird als Signal von horizontal und vertikal ausgerichteten (nicht dargestellten) Antennen der Interaktionsvorrichtung 220 empfangen.

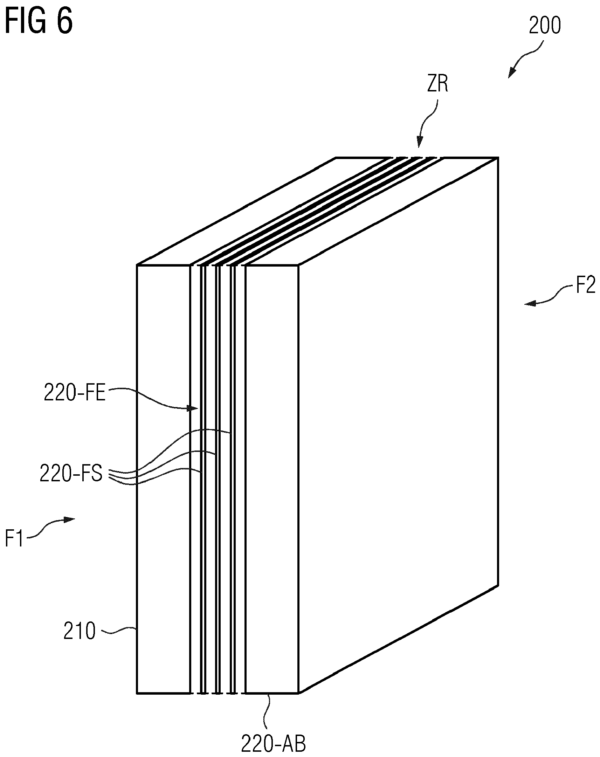

Selbstverständlich können die Elemente von Interaktionsvorrichtungen 220 gemäß anderer Ausführungsformen mit der Ausführungsform von Figur 6 kombiniert werden. So kann die in Figur 6 gezeigte Ausführungsform eine oder mehrere Bedienfelder 220-BF aufweisen, die in das Abdeckelement 220-AB eingearbeitet bzw. auf diesem angeordnet sind. Ferner kann das Abdeckelement 220-AB auch die Infrarot-Licht emittierende Leuchtelemente 220-LE sowie entsprechende Sensorelemente 220-SE aufweisen, die entsprechend der in Figur 4 gezeigten Ausführungsform an den Kanten des Abdeckelements 220-AB angeordnet sind. Insbesondere kann eine solche Ausgestaltung mit Leucht- und Sensorelemente 220-LE, 220-SE mit einer LCD-, OLED- oder TFT-Schicht als Funktionsschicht 220-FS kombiniert werden.

Gemäß einer weiteren nicht gezeigten Ausführungsform kann das Abdeckelement 220-AB auch ohne ein zwischen-geschichtetes Funktionselement 220-FE direkt an dem Glaselement 210 angeordnet bzw. angebracht sein. Eine Berührungserkennung kann dann z.B. optisch durch an dem Abdeckelement 220-AB angeordnete Leucht- und Sensorelemente 220-LE, 220-SE oder Kamerabasiert erfolgen.

In einer weiteren, nicht gezeigten Ausführungsform kann das Funktionselement 220-FE unter Weglassung des Abdeckelements 220-AB in dem Glaselement 210 eingebettet sein. Mit anderen Worten wäre das Funktionselement 220-FE in das Glaselement 210 eingeschichtet und - von Anschlüssen zur Stromversorgung bzw. zum Datenaustausch abgesehen - von dem Glaselement 210 umgeben.

Das Abdeckelement 220-AB und das Glaselement 210 können beispielsweise durch Verkleben aneinander befestigt sein. Vorzugsweise kann ein Verkleben an den Rändern bzw. in den Randbereichen von Abdeckelement 220-AB und Glaselement 210 erfolgen. Alternativ kann ein flächiges Verkleben erfolgen - insbesondere dann, wenn kein Funktionselement 220-FE vorhanden ist.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Strahlenschutzvorrichtung (200) zur Abschirmung eines Nutzers (N) vor ionisierender Strahlung, umfassend:
- ein Glaselement (210), das als Schutzglas gegen die ionisierende Strahlung ausgebildet ist, und
- eine Interaktionsvorrichtung (220) zum Empfangen einer Nutzereingabe durch den Nutzer (N),
- eine Freigabevorrichtung (270) zum Empfangen einer Freigabeeingabe durch den Nutzer (N), und
- wobei die Interaktionsvorrichtung (220) und/oder die Freigabevorrichtung (270) wenigstens abschnittsweise transparent ausgebildet ist und wenigstens einen Teil einer Fläche des Glaselements (210) überdeckt.

2. Strahlenschutzvorrichtung (200) nach Anspruch 1, ferner umfassend eine Schnittstelle (240) zur Bereitstellung eines Steuersignals und ein Steuermodul (260), wobei die Interaktionsvorrichtung (220) zur Bereitstellung der Nutzereingabe datentechnisch mit dem Steuermodul (260) verbunden ist, wobei die Freigabevorrichtung (270) zur Bereitstellung der Freigabeeingabe datentechnisch mit dem Steuermodul (260) verbunden ist, wobei das Steuermodul (260) ausgebildet ist, das Steuersignal an der Schnittstelle (240) auszugeben, wenn die Nutzereingabe und die Freigabeeingabe gleichzeitig oder innerhalb eines zeitlichen Sicherheitsfensters erfolgen und/oder empfangen werden.

3. Strahlenschutzvorrichtung (200) nach einem der vorherigen Ansprüche, **dadurch gekennzeichnet, dass** die Interaktionsvorrichtung (220) eine erste Sensorfolie als Interaktionsschnittstelle für den Nutzer (N) umfasst, wobei die erste Sensorfolie zur Eingabe und/oder zum Empfangen der Nutzereingabe durch den Nutzer (N) ausgebildet ist.

4. Strahlenschutzvorrichtung (200) nach einem der vorherigen Ansprüche, **dadurch gekennzeichnet, dass** die Freigabevorrichtung (270) eine zweite Sensorfolie als Interaktionsschnittstelle für den Nutzer (N) umfasst, wobei die zweite Sensorfolie zur Eingabe und/oder zum Empfangen der Freigabeeingabe durch den Nutzer (N) ausgebildet ist.

5. Strahlenschutzvorrichtung (200) nach Anspruch 3 oder 4, **dadurch gekennzeichnet, dass** die erste Sensorfolie und/oder die zweite Sensorfolie eine Kraftmessfolie zur Erfassung einer einwirkenden Kontaktkraft (KF) bildet, wobei die Kraftmessfolie aufweist:
- eine Deckschicht zur Aufnahme der Kontaktkraft (KF),
- eine Anbringschicht, mit der die Kraftmessfolie auf einer Fläche angebracht ist oder anbringbar ist, sowie
- eine Sensorschicht, die derart ausgebildet ist, dass
- sich ein elektrischer Widerstand der Sen-sorschicht als Funktion der auf die Kraftmessfolie einwirkenden Kontaktkraft (KF) ändert, und
- wenigstens ein auf dem elektrischen Wider-stand basierendes Messsignal abgegriffen werden kann.

6. Strahlenschutzvorrichtung (200) nach Anspruch 5, **dadurch gekennzeichnet, dass** das Steuermodul (260) ausgebildet ist, als das Steuersignal ein auf dem abgegriffenen Messsignal und/oder der einwirkenden Kontaktkraft (KF) basierendes Steuersignal auszugeben.

7. Strahlenschutzvorrichtung (200) nach einem der vorherigen Ansprüche, **dadurch gekennzeichnet, dass** Interaktionsvorrichtung (220) und/oder die Freigabevorrichtung (270) eine Berührungserkennung implementiert, die ausgewählt ist aus:
- einer optischen Berührungserkennung,
- einer kapazitiven Berührungserkennung,
- einer induktiven Berührungserkennung,
- einer resistiven Berührungserkennung, und/oder
- Kombinationen der vorgenannten Berührungserkennungen.

8. Strahlenschutzvorrichtung (200) nach einem der vorherigen Ansprüche, **dadurch gekennzeichnet, dass** die Interaktionsvorrichtung (220) in einem Eingabeabschnitt auf dem Glaselement (210) angeordnet ist und die Freigabevorrichtung (270) in einem Freigabeabschnitt auf dem Glaselement (210) angeordnet ist, wobei der Eingabeabschnitt und der Freigabeabschnitt beabstandet angeordnet sind.

9. Strahlenschutzvorrichtung (200) nach Anspruch 8, **dadurch gekennzeichnet, dass** das Glaselement (210) eine flächige Frontseite und eine flächige Rückseite aufweist, wobei der Eingabeabschnitt auf der Frontseite angeordnet ist und der Freigabeabschnitt auf der Rückseite angeordnet ist.

10. Strahlenschutzvorrichtung (200) nach Anspruch 8 oder 9, **dadurch gekennzeichnet, dass** der Eingabeabschnitt und/oder der Freigabeabschnitt in einem Randbereich des Glaselements (210) angeordnet ist.

11. Strahlenschutzvorrichtung (200) nach einem der vorherigen Ansprüche, **dadurch gekennzeichnet, dass** die Interaktionsvorrichtung (220) ein oder mehrere vorgefertigte Bedienfelder zur Eingabe einer Nutzereingabe umfasst.

12. Strahlenschutzvorrichtung (200) nach einem der vorherigen Ansprüche, **gekennzeichnet durch** eine Anzeigevorrichtung (250) zur Anzeige des Erhaltens der Nutzereingabe, des Erhaltens der Freigabeeingabe und/oder der Ausgabe des Steuersignals.

13. Strahlenschutzvorrichtung (200) nach einem der vorhergehenden Ansprüche, bei der die Interaktionsvorrichtung (220) umfasst:
- ein Abdeckelement (220-AB), das parallel zum Glaselement (210) angeordnet ist,
- ein Funktionselement (220-FE), das zwischen Abdeckelement (220-AB) und Glaselement (210) angeordnet ist und zum Darstellen von Daten für den Nutzer (N) und/oder zum Empfangen einer Nutzereingabe durch den Nutzer (N) ausgebildet ist.

14. Medizinische bildgebende Modalität (100) mit einer Strahlenschutzvorrichtung (200) nach einem der vorherigen Ansprüche.
